(19) 

**Europäisches Patentamt**
**European Patent Office**
**Office européen des brevets**

(11) **EP 4 362 805 B1**

(12) **EUROPEAN PATENT SPECIFICATION**

(45) Date of publication and mention
of the grant of the patent:
**10.06.2026  Bulletin 2026/24**

(21) Application number: **22734642.6**

(22) Date of filing: **24.06.2022**

(51) International Patent Classification (IPC):
**A61B 7/00** (2006.01)

(52) Cooperative Patent Classification (CPC):
**A61B 7/003**

(86) International application number:
**PCT/EP2022/067429**

(87) International publication number:
**WO 2023/274894 (05.01.2023 Gazette 2023/01)**

(54) **METHOD FOR MONITORING ACOUSTIC EVENTS IN A PATIENT**

VERFAHREN ZUR ÜBERWACHUNG VON AKUSTISCHEN EREIGNISSEN BEI EINEM PATIENTEN

PROCÉDÉ DE SURVEILLANCE D'ÉVÉNEMENTS ACOUSTIQUES CHEZ UN PATIENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.07.2021  EP 21183483**

(43) Date of publication of application:
**08.05.2024  Bulletin 2024/19**

(73) Proprietor: **CSS Versicherung AG**
**6002 Luzern (CH)**

(72) Inventors:
 • **SHIH, Chen-Hsuan**
 **8003 Zürich (CH)**
 • **DA CONCEIÇÃO BARATA, Vitor Filipe**
 **8047 Zürich (CH)**
 • **OTT, Kai Robin**
 **3013 Bern (CH)**
 • **TINSCHERT, Peter Jan**
 **8006 Zürich (CH)**

(74) Representative: **KATZAROV S.A.**
**Geneva Business Center**
**12 Avenue des Morgines**
**1213 Petit-Lancy (CH)**

(56) References cited:
**WO-A2-2006/117780      US-A1- 2006 074 334**
**US-A1- 2015 245 788**

**Description**

**Technical Field**

The present invention relates to a computer implemented method for detecting a patient's acoustic events, for instance coughs, from audio data of the patient, and to a device comprising means for carrying out said method.

The invention further relates to a computer program comprising instructions, which, when the program is executed by a computer, cause the computer to carry out the method according to the present invention.

**Background of the art**

Asthma is one of the most prevalent chronic respiratory diseases. Despite increased investment in treatment, little progress has been made in the early recognition and treatment of asthma exacerbations over the last decade.

Nocturnal cough monitoring may provide an opportunity to identify patients at risk for imminent exacerbations. Recently developed approaches enable smartphone-based cough monitoring by using the microphone of the smartphone to record the cough.

Subsequently the audio file is processed to detect cough event, notably for eliminating the noise or other non-relevant audio events, and to count the cough event for instance overnight.

An example of approach is described in the document Barata et al, entitled "Automatic Recognition, Segmentation, and Sex Assignment of Nocturnal Asthmatic Coughs and Cough Epochs in Smartphone Audio Recordings: Observational Field Study", J.Med. Internet Res, vol.22, issue 7, 2020.

However, this approach is not practical for real-time analysis on mobile devices, notably because it requires more computational power than is present in mobile devices, for instance it requires the computational power of a supercomputer, to process the audio files recorded by the mobile device.

Additionally, it requires a lot of storage on the smartphone to record the entire nights of audio file. After recording, a considerable amount of raw audio data must be uploaded to a server (e.g., the Euler supercomputer), which raises data privacy concerns and requires patients to have a fast and reliable internet connection.

Eventually, patients have to wait for results until data upload and analysis on a server to be completed which generates multiple days of delay between recording and receiving results. Prior art document US 2015/245788 discloses a computer-implemented method for detecting a patient's acoustic events according to the preamble of independent claim 1.

Therefore, there is a need to provide a solution to facilitate the monitoring of acoustic event and overcome the issues of the existing approaches.

**Summary of the invention**

The above problems are solved or at least minimized by the present invention, which is defined in the appended claims.

This object has been achieved by providing a computer implemented method for detecting a patient's acoustic events, for instance coughs, from audio data of the patient during an analyzing period, the method comprising:

- a recording step to record an audio signal from the patient by using a mobile device comprising recording means, and
- a processing step to process the recorded audio signal into audio data by using a processing module embedded in the device,
- a detection step to detect said acoustic events from the audio data,

characterized in that the method further comprises a monitoring step, before the recording step, to select segments of a determined duration of the patient's audio signal to be recorded on the device, said monitoring step comprising:

- applying an adaptive loudness threshold named ALT on the patient's audio signal to identify sounds that exceed said adaptative loudness threshold named relevant sounds,
said adaptative loudness threshold being based on an initial loudness threshold that is configured to be adjusted continuously during said analysing period, and recording on the device the segments of patient's audio signal located downstream said identified relevant sounds.

Another object of the present invention is to provide a computer implemented method for assigning a detected acoustic event, for instance coughs, to a patient comprised in a group of patients, the method comprising:

- Providing a recorded segment of audio signal of one of the patients from the group;

- Detecting an acoustic event in each of the patients from the group by using a method according to the present invention;

- Comparing the detected acoustic event with the provided recorded segment of audio signal;

- If there is a match between the detected acoustic event and the provided recorded segment audio signal, assigning the detected acoustic event to said patient.

A further object of the present invention is to provide device comprising means for carrying out the method of the present invention, preferably the device is chosen among a mobile device, for instance a smartphone.

A further object of the present invention is to provide a computer program comprising instructions, which, when the program is executed by a computer, cause the computer to carry out the method of the present invention.

**Description of the invention**

The invention concerns a computer-implemented method for detecting a patient's acoustic events, for instance coughs, from audio data of the patient during an analyzing period, the method comprising:

- a recording step to record an audio signal from the patient by using a mobile device comprising recording means, and

- a processing step to process the recorded audio signal into audio data by using a processing module embedded in the device,

- a detection step to detect said acoustic events from the audio data,

characterized in that the method further comprises a monitoring step, before the recording step, to select segments of a determined duration of the patient's audio signal to be recorded on the device, said monitoring step comprising:

- applying an adaptive loudness threshold named ALT on the patient's audio signal to identify sounds that exceed said adaptative loudness threshold named relevant sounds,
said adaptative loudness threshold being based on an initial loudness threshold that is configured to be adjusted continuously during said analysing period, and recording on the device the segments of patient's audio signal located downstream said identified relevant sounds.

The logic behind the present invention is that patients tend to cough (example of acoustic event) rather infrequently during a period of time, for instance during the night. Therefore, it is possible to vastly limit the number of computations needed to detect an acoustic by excluding or discarding as many parts of the night for instance as possible in which no acoustic event occurs (i.e., patients most likely do not cough). If the method according to the present invention is executed only at times when an acoustic event does occur (when patients cough), namely for relevant sounds, a vast majority of computations can be spared without scarifying the accuracy of the detection since the most relevant part of the audio signal will be analysed. This approach allows to limit the computation power required to execute the detection process.

Advantageously, the method, for instance, the computer program or software related thereof, can be run on a smartphone since computation and analysis are limited to the most relevant part of the signal, namely relevant sounds. In other words, all the necessary calculations can be performed by using exclusively the computational power of a smartphone.

The present invention also allows to limit the storage capacity required on the mobile device executing the computer implemented method since it does not require a continuous recording of the audio signal or audio data.

In general, if the threshold is high, fewer acoustic events will be recorded and analyzed, but a relatively higher number of acoustic events could not be detected if they are too silent. For a low threshold a higher number of coughs can be detected, but the computational resources (CPU, memory, power) usage will increase, since more acoustic events need to be analyzed. Moreover, the threshold can vary from patient to patient and/or from one analysing period to another (night to night) for various reasons (e.g., different cough loudness, bedroom outline, smartphone placement, background noise). Therefore, setting a static threshold, that is the same for every patient, is not adequate. In the present invention, an adaptive loudness threshold is used. The point of this adaptive loudness threshold is to find the most suitable loudness threshold per patient and per analysing period (per night for instance night).

Advantageously, the present invention offers an adaptive threshold that aims at finding the ideal loudness threshold per patient, and also per recording period (or recording session) for a same patient (i.e. per night or day).

In the recording step of the present invention, an adaptative loudness threshold is used to assess whether a segment of the audio signal is relevant, i.e., relevant sound, (likely to contain an acoustic event) or not. An initial loudness threshold is set as a basis or reference loudness threshold. The loudness threshold is adaptive, meaning that the loudness threshold is adjusted continuously to personalize the threshold to the patient and the patient's located environment. In other words, the initial loudness threshold is adjusted depending on the sound level of the acoustic events (i.e., retroactively) in the patient during the recording or while the signal is recorded.

Advantageously, the adaptative loudness threshold is patient-specific and/or environment-specific, meaning that the loudness threshold is adjusted to respectively the audio signal of each patient and each environment allowing a personalized recording.

Advantageously, the audio signal having a sound level below the adaptative threshold is not recorded, which allows keeping the storage capacity of the mobile device for relevant sounds. This allows to limit the storage capacity required on the device.

Additionally, when a relevant sound detected, the recording is initiated to record the portion of the audio file, namely a segment, downstream or immediately after or next to the detected relevant sound. In other words, the detection of a relevant trigger the recording of the segment of a determined duration.

Contrary to the existing approaches, the present invention avoids processing the audio signal continuously for sound below a loudness threshold but rather aims at processing discrete or chosen portions of the audio file exceeding the adaptative threshold.

The adaptative loudness threshold permits to record overall less audio signal, which means that less data needs to be analysed and thus computational resources are spared. The audio file selections are made without sacrificing on the accuracy or with a very limited or negligible effect on the accuracy of the acoustic event detection. The reason is that only or mostly irrelevant parts of the audio signal are excluded in which there is no acoustic event or in which the chance to have an acoustic event is very low or negligible.

Preferably, the analyzing period is variable. For instance, the analyzing period can be chosen by the patient, who can start and stop the monitoring manually.

The analyzing period is preferably comprised between about 4 hours and about 14 hours, preferably between about 6 hours and 12 hours, for instance 8 hours or 12 hours.

The analyzing period could be started when the patient goes to sleep and stopped when the patient gets up, for instance on average 8 hours.

The analyzing period could be chosen to be at least 1 second and not restricted in length, more preferably between 1 second and 24 hours, for instance 12 hours.

According to the invention as defined in independent claim 1, the adaptative loudness threshold is adjusted based on at least three parameters defined as:

- a first parameter named T and based on the loudness of the detected acoustic events from the patient;
- a second parameter named T_N and based on a factor M times of the average loudness of the audio signal;

- a third parameter named T_min and based on a determined minimum threshold;

wherein said adaptive loudness threshold ALT corresponds to the maximum value chosen among the first parameter, the second parameter and the third parameter and is defined as

$$ALT = max\ (T,\ T\_min,\ T\_N)$$

In other words, during the analyzing period, the threshold varies and corresponds to one the parameters, namely either to the first parameter, to the second parameter, or to the third parameter.

For example, if the first parameter is -24.8dB, the second parameter is -38dB and the third parameter is -32dB, the adaptative loudness threshold corresponds to the first parameter, that is -24.8dB. The maximum of negative numbers is the least negative.

Preferably, the first parameter T is based on the distribution of the acoustic event detected in the patient, said distribution being adjusted continuously as the acoustic events are detected. Thus, the adaptative loudness threshold can adjust to the loudness of the patients' cough, room size, distance to phone, orientation etc.

Preferably, the factor M time of the second parameter T_N is chosen between 0 and about 100, preferably about 2 and about 50, more preferably between about 4 and about 25, in particular between about 8 and about 12.5, for instance between about 9 and about 11, for instance 10. Thus, in a very noisy environment, the number of acoustic event (cough for instance) discarded or sacrificed is very limited (typically a few) to analyze way fewer audio signals (that would be mostly caused by loud noise).

Preferably, the minimum threshold of the third parameter is comprised between about -18dB and about -45dB, preferably between about -22dB and about -42dB, more preferably between about -27dB and about -37dB, in particular between about -30dB and about -34dB, for instance between about -31dB and about -33dB, for instance about -32dB. Thus, if the acoustic events (coughs for instance) that can be monitored or recorded are really silent (maybe the patient is too far from the recording means), the threshold is not lowered indefinitely, which would induce the analysis of silent (weak) audio signals.

Preferably, the determined duration of the segment is comprised between about 1 second and about 20 seconds, preferably between about 2 seconds and about 15 seconds, more preferably between about 3 seconds and about 10 seconds, in particular between about 4 seconds and about 8 seconds, for instance between about 5 seconds and about 7 seconds, for instance about 6 seconds, for instance between about 5 seconds and about 8 seconds, for instance about 6.5 seconds.

Advantageously, the duration of the segment is chosen to limit the impact on the storage capacity of the device. Preferably, the duration of a segment is in seconds, is comprised between about 1 second and about 20 seconds, preferably between about 2 seconds and about 15 seconds, more preferably between about 3 seconds and about 10 seconds, in particular between about 4 seconds and about 8 seconds, for instance between about 5 seconds and about 7 seconds, for instance about 6 seconds.

In a preferred embodiment, the method further comprises, before the monitoring step, a status check step comprising:

- determining if the device is in use or not in use based on at least one sensor of the device, and starting the monitoring step if the device is not in use; and/or

- determining if the device has an energy level above a predetermined level, and starting the monitoring step if the energy level is above the predetermined level;

The status check filter is particularly adapted for detecting acoustic events when the patient is asleep, for instance during nighttime. The logic of the status check filter is that the status of the mobile device is correlated with the state of the patient. In other words, if the device is in use, the patient is not asleep. Determining the status of the device allows execution of the method only when the patient is asleep.

The status check filter can also check it the battery level is sufficient to execute the method of the present invention, i.e., above a predetermined energy level. The point is for instance to avoid that the execution of the method of the present invention drain the entire battery and prevent the execution of other actions or feature on the mobile device, for instance, an alarm if the device is a smartphone. For instance, the predetermined energy level is between about 10% and about 40 %.

Preferably, status check is based on sensors installed in the device capable of giving information on the position of the device, for instance an accelerometer, and/or on the status of the device for instance, screen on/off and brightness, battery status.

In a preferred embodiment, the processing step comprises a pre-processing step that comprises:

- Applying a peak detection filter on said recorded segments to extract a determined number of peaks;

- Processing the extracted peaks of the patient's audio signal to provide the audio data;

The peak filter aims at identifying distinct peak(s) or acoustic peak(s) within the recorded segments instead of processing the entire whole audio file or the computationally expensive sliding window logic of existing approaches such as Barata et al (cf para.[006] above). In the present case, only the extracted peaks of the audio file are processed to provide the audio data, for instance audio data as Mel spectrograms.

This allows saving computational resources without sacrificing the overall accuracy in a meaningful way since the computational resources process the relevant sounds.

For example, the audio data that can be used in the present invention as a soundwave could be:

- kept in the format it is in,

- or processed to a normal spectrogram,

- or processed to a Mel spectrogram,

- or discretely transformed to Fourier space,

- or converted to an image of the soundwave,

- or transformed with the Discrete Wavelet Transform,

The number of peak detectable per recorded segment is adaptive and is determined depending on the processing module specifications, for instance, the processing speed or the calculation power of said processing module, or the acoustic environment. This allows seamless execution of the peak finder depending on the computation power of the device.

The peak filter can be optimized depending on some setting of the device, for instance, the availability of the device's computational power and battery. For devices which have limited open resources (i.e. availability of computational power and battery), there is an peak upper limit per X second(s) (X being a determined number), for instance 10 peaks per 6 seconds. For devices which do not have limited open resources (i.e. availability of computational power and battery), there is no upper limit per X second(s) (X being a determined number).

For instance,

- in a device whose processing unit is not fully in use, the number of peaks per 6 seconds can be increased,

- in a device whose processing unit is fully in use, the number of peaks per 6 seconds can be decreased,

- and where the default is between around 3 peaks / 6 seconds and around 12 peaks per 6 seconds, for instance 6 peaks per 6 seconds for devices which have limited open resources.

In a preferred embodiment a minimal distance is set between each peak, preferably personalized to each patient, that is comprised between about 0.2 seconds and about 1.1 seconds, preferably about 0.2 seconds and about 0.8 seconds, more preferably between about 0.2 seconds and about 0.40 seconds, in particular about 0.35 seconds. This prevents a peak, and hence an acoustic event, from being counted multiple times. The inventors found out that generally only less than 5% of peaks are closer to each other than .35s. This also allows for a higher accuracy of the present invention in comparison to applying a peak finder without this boundary condition.

In another embodiment, the peak filter comprises :

- Calculating the power (i.e. the sound level) of non-overlapping time frames of Y samples per segment (Y being a determined number), for instance 1500 samples for a 6.5 seconds segment (93.75 ms @ 16 kHz), for instance as the root mean square of those samples;

- If a power of a frame is above a certain threshold (for instance -50 dB), the power of said frame is compared to the power of the two preceding frames;

- If there is an increase in power of a certain threshold (for instance -5 dB) (i.e. if the discrete curvature estimate is convex), the frame is considered as a frame of interest;

- In each frame of interest, the peaks correspond to the highest amplitude;

In this embodiment, the peak filter provides an improved peak detection (i.e. acoustic event counting) when multiple successive acoustic event, for instance cough, occur consecutively in a segment.

Preferably, the processing step comprises a pre-processing step that comprises:

- Applying at least one preprocessing filter on the recorded segments of audio signal to provide a selection of segments, preferably said preprocessing filter being chosen from the list comprising entropy filter, voice activity detection (VAD), preferably an entropy filter followed by a voice activity detection (VAD);

- Applying a peak finder on said selected segments to extract a determined number of peaks within each selected segment;

The preprocessing filter permits to exclude segments without having to compute any audio data, such as Mel spectrogram for them, which is a computationally demanding task. In this way, only segments that have likely relevant acoustic events in them are processed.

In a preferred embodiment, the detection step comprises applying an ensemble of at least one trained inference model on said recorded segment of the audio data of the patient as an input to compute a probability as an output to detect an acoustic event.

The ensemble can comprise one or more inference model.

In an embodiment, said ensemble comprising one trained inference model, the detection step comprises;

i) Applying said inference model on said recorded segment of audio data of the patient as an input, to compute a probability as an output, said audio data contains at least one acoustic event,

ii) if the probability is inferior to a reference value the acoustic event is rejected, whereas if the probability is superior to the first reference value the acoustic event is validated;

The ensemble can comprise one or more trained inference models :

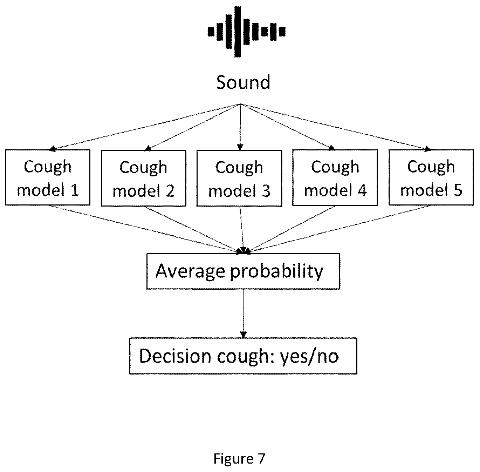

- An ensemble of at least two trained inference model being configured for detecting a same type of acoustic event, for instance cough; preferably, the output probability is the mean probability of said at least two inference model as shown in figure 7 (i.e. two step approach described below); in other words, multiple trained inference models vote on the same task; or

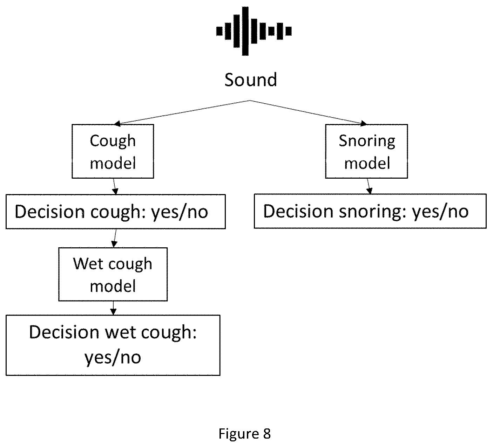

- An ensemble of trained inference model, at least one, each inference model being configured for detecting a type of acoustic event, for instance snoring and coughing; each trained inference model is applied in parallel on the recorded segment, as shown on figure 8; in other words, each trained inference model votes on a specific task; several trained inference model can be used successively, as represented in figure 8 where the "wet cough" model is followed by the "cough model".

In an embodiment, the detection step comprises

- i) Applying a first trained inference model on said recorded segment of the audio data of the patient as an input, to compute a probability as an output,

said audio data contains at least one acoustic event,

said first trained inference model belonging to an ensemble of trained inference models comprising "n" trained inference models, "n" superior or equal to two, to detect said acoustic events; and

if the probability is inferior to a first reference value, the acoustic event is rejected, whereas

if the probability is superior to the first reference value, step i) is repeated with the next trained inference model from said ensemble comprising "n" trained inference model; and

- ii) the acoustic event is validated if the probability is superior to the first reference value for all the "n" trained models, and if there is a mean probability for all the "n" trained inference model of the ensemble that is above a second reference value;

Advantageously, the ensemble of trained inference models is used to detect the acoustic event in the audio data. For instance, said ensemble is used to derive output from the inference models that process the Mel spectrograms.

The recorded segment of audio data is used as input to an ensemble of trained inference models to compute a probability and a mean probability as output. Derivative of said recorded segment can also be used as input such as Mel spectrograms. Depending on these computed probabilities, the acoustic event is validated (i.e., counted) or rejected (i.e., not counted).

Advantageously, a two steps approach is used herein based on two reference values, namely a first reference value and a second reference value. Overall, the point is to obtain a mean probability above a second reference value to have an acoustic event validated.

The mean probability of all the trained inference models depends on each probability of each trained inference model. Therefore, in the first step, one of the trained inference models is executed to compute a probability. The probability computed by the first trained inference model is used to determine if the remaining trained inference model of the ensemble should be executed or not. In other words, the first computed probability computed by the first trained inference model determines if the rest of the trained model shall be executed or not. If the first computed probability is inferior to a first reference value, the mean probability for all the trained inference models cannot be above the second reference value, so that there is no need to execute said remaining trained inference model.

For instance, let assume the ensemble of trained inference models comprises 5 (five) trained inference models, the first reference value is set to 75 % and the second reference value set to 95%:

- If the probability computed by the first trained inference model is below 75%, the mean probability of 95% cannot be reached any more; there is no need to execute the remaining 4 (four) trained inference models and the audio data corresponding to said peak is not counted as acoustic event.

- If the probability computed by the first trained inference model is above 75%, the mean probability of 95% can be reached; the remaining 4 (four) trained inference models are executed and if the mean probability of all the ensemble of trained inference model is above 95%, the audio data corresponding to said peak is counted as acoustic event.

The point of these two steps' approaches herein save the computational power without sacrificing any accuracy.
Preferably, the first reference value is correlated with the mean probability for all the inference models of the ensemble and the number "n" of trained inference models by using the following formula:

$$\text{First reference value} = 1 - ((1 - \text{second reference value}) \times \text{number "n"})$$

Preferably the trained inference model is chosen among a neural network classifier.
Advantageously, it is possible to use specific trained inference model configured and trained for detecting specific acoustic events named type of acoustic event, such as daytime acoustic event or cough, dry or wet cough, wheeze cough.
Preferably, each of the "n" trained inference model of the ensemble is dedicated to the detection of a specific type of acoustic event. This allows to customize the ensemble of trained inference model depending on the intended use and target user population of the application. It allows for instance to serve different use cases by combining the detection of different symptoms. There are diseases for which cough does not play a role, but snoring does, so having the option to combine trained inference models in our pipeline increases the applicability of the present invention.
In an embodiment, at least one of the "n" trained inference models of the ensemble is dedicated to the detection of a type of patient's acoustic events, for instance daytime acoustic event, dry or wet cough, wheeze cough, snoring, specific breathing patterns.
In an embodiment, the "n" trained inference models comprise a model configured for detecting acoustic event during daytime. The inventors discovered that the acoustic events (i.e.noise and sounds) that do not occur during sleep of the patient but during daytime (e.g., laughing and cutlery sound) could lead to high false-positive rates from the trained models which have not been trained for day time acoustic event detection. Thus, it is advantageous to use an additional model specifically trained and configured for day time acoustic event detection to remove or limit the false positive.
Preferably, the daytime trained inference model is trained by transfer learning using available sound classifier, such as pre trained deep neural network, for instance Yamnet from Google™. Additionally, (or alternatively but preferably additionally on top of the training by transfer learning), the daytime trained inference model is trained by using as input the acoustic events wrongly classified by the rest of the "n" trained inference ensemble such that the daytime trained inference model learns how to filter the false positive acoustic event.
Preferably, the "n" ensemble comprises at least one trained inference model for nighttime, for instance five, and one trained inference model for day time.
Preferably, at least one of "n" trained inference models of the ensemble is dedicated to the detection of daytime acoustic event.
Preferably, at least one of "n" trained inference models of the ensemble is dedicated to the detection of nighttime acoustic event.
Preferably, at least one of "n" trained inference models of the ensemble is dedicated to the detection of nighttime acoustic event and at least one of "n" trained inference models of the ensemble is dedicated to the detection of daytime acoustic event.
Preferably, the nighttime refers to a time period when the patient is asleep while the daytime refers to a time period when the patient is awake.
In another embodiment, the "n" ensemble of trained inference model comprises one trained inference model, preferably said trained inference model being trained for detecting acoustic event in daytime and in night time. In other words, a unique trained inference model allows to determine whether an acoustic event shall be counted or discarded, for instance whether the acoustic event is a cough or not, regardless of whether this has occurred during day or night.
Advantageously, in one embodiment the ensemble of trained inference models can comprise a family of trained inference models (at least two trained inference models)

In a preferred embodiment, the ensemble of trained inference models used in the present invention is described in the document Barata et al, entitled "Automatic Recognition, Segmentation, and Sex Assignment of Nocturnal Asthmatic Coughs and Cough Epochs in Smartphone Audio Recordings: Observational Field Study", J.Med. Internet Res, vol.22, issue 7, 2020.

Preferably, the first reference value comprised between about 50 % and 100 %, preferably between about 60% and about 90 %, more preferably between about 70% and 80%, for instance about 75 %.

Preferably, the second reference value is comprised between about 50 % and 100 %, preferably between about 70% and about 100 %, more preferably between about 80% and 100%, more preferably between about 90 % and 100 %, for instance about 95 %.

Preferably, the patient is comprised in a group of "p" patients comprising at least two patients, the method further comprising an assigning step for assigning a detected acoustic event to a patient from the said group, the assigning step comprising:

- Applying a computational tool, for instance a statistical model on the detected acoustic event, said computational tool being configured to assign a detected acoustic event to a predetermined patient;
- Attributing the detected acoustic event to one patient from the group if there is a correlation between said computation tool and the detected acoustic event;

The computational tool can be a trained inference model or a machine learning model such as a Neural Network or Random Forest classifiers. The computational tool can also be a statistical model.

Preferably, the computation tool has been trained to assign a detected acoustic event to a type of patient is based on gender, for instance male or female, the age for instance child or adult. In other words, the predetermined patient is for instance a male or a female, a child or an adult. Advantageously, it is possible to have one computation tool, for instance pre-trained model for each type of predetermined patient so as to assign to a specific type of patient. There is no need to have a sample of previously detected acoustic event for each patient whose audio data is analyzed.

Alternatively, the computation tool has been trained to assign a detected acoustic event to a designated patient based on previously detected acoustic event from said designated patient. In other words, the predetermined patient is one specific patient for who acoustic events have been already detected. The previously detected event will be used as a template to assign the acoustic event to this specific patient. Advantageously, the assigning step allows to assign coughs to a patient not based labels like male or female, but by comparing a detected acoustic event with a patient acoustic event that was recorded earlier. Additionally, it is possible to assign acoustic event to patient, even if they are of the same group (for example with a same gender). For instance, when a female patient coughs, it is possible to assign that cough to her even if she shares the room with another female person who coughs.

The assigning step can be used in a group of patients. It can also be used for one patient. This is advantageous for instance if the gender of the patient is unknown, if the age of the patient is unknown, if the identity of the patient is unknown.

When an acoustic event is detected, a computation tool (for instance a model or statistical model) will be used to label it or assign it to a predetermined patient or predetermined type of patient, for instance a male or a female. Knowing the gender of the patients from the group of patients, it is possible to know if the acoustic event is of the patient (genders match) or of their partner. Alternatively, a model can label or assign a detected acoustic event as "adult cough" or "child cough" for example, which would help to assign coughs to a patient, if the e.g., adult patient is in the same room as a child who also coughs.

The particular advantages of the methods are similar to the ones of the device of the invention and will thus not be repeated here.

The embodiments describe for the computer-implemented method also apply to the computer program and to the device according to the present invention mutatis mutandis.

## Brief description of the drawings

Further particular advantages and features of the invention will become more apparent from the following non-limitative description of at least one embodiment of the invention which will refer to the accompanying figures (tables and plots), wherein

- Figure 1 is a description of the method for detecting a patient's acoustic events, here coughs according to a first embodiment of the present invention;
- Figure 2 is a description of the method for detecting a patient's acoustic events, here coughs according to a second embodiment of the present invention;
- Figure 3 represents an embodiment to continuously adjust the adaptive loudness threshold according to the present invention;

- Figures 4,5,6 represent the evolution of the adaptative loudness threshold in three examples - respectively example 1, example 2 and example 3 - of the method according to the present invention;
- Figures 7 and 8 represent two configurations of ensemble of trained inference models;

## Detailed description of the invention

The present detailed description is intended to illustrate the invention in a non-limitative manner since any feature of an embodiment may be combined with any other feature of a different embodiment in an advantageous manner.

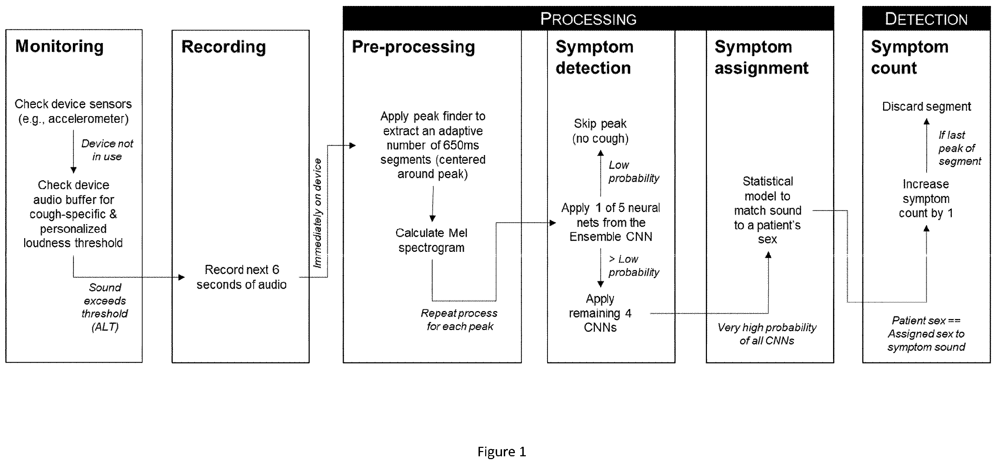

In the examples represented in figures 1 to 6, the mobile device used in the present invention is a smartphone and all the calculations required to run the method are performed by using the computation resources of the smartphone. Additionally, in the examples represented in figures 1 to 5, the acoustic event that is detected is cough. However, the present invention is not limited to smartphones as mobile devices or cough as the only detectable acoustic event.

Figure 1 is a scheme illustrating an embodiment of the method for detecting patient's cough from audio data during an analyzing period of 8 hours during the night (patient asleep) for instance in the present embodiment.

The method comprises four main steps, namely a monitoring step, a recording step, a processing step and a detection step.

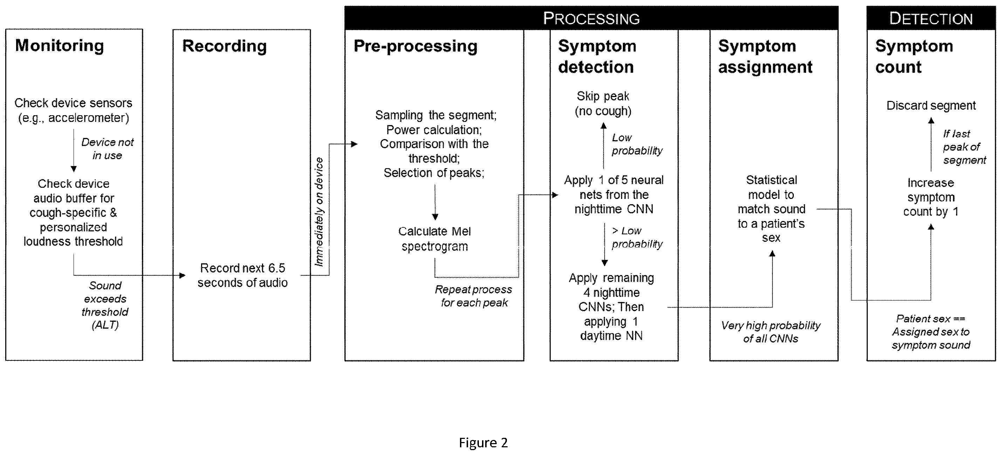

The first step is the monitoring step which aims at assessing whether a specific segment is relevant. To that end, the device checks the audio buffer continuously for noises that exceed an adaptative loudness threshold (to be discussed here above with figure 2). The monitoring step permits to select segments of a determined duration of the patient's audio signal to be recorded on the device. In the present example, the determined duration is 6 seconds.

The monitoring steps also comprise a status check step to check continuously (for instance every 10ms) whether the device is currently in use based on other sensors (i.e., accelerometer, screen brightness, and battery status). Only if the device is not in use and has a sufficient battery level, the method (i.e. detection pipeline) is executed. The innovative improvement of this step is two-fold: 1) it intends to analyze nocturnal cough (i.e., cough in the nighttime is defined through the sleep state of a patient, and if the device is in use, the patient is not asleep) and 2) it prevents impairment of patients' user experience when using their device for other purposes than cough detection (if the pipeline is executed it requires computational resources which then lack for the seamless execution of other smartphone applications; if battery state is not considered, the detection pipeline may drain the entire battery, so that an alarm to wake up the patient in the morning would not be executed).

During the monitoring step, when a sound of the audio data exceeds the adaptative loudness threshold (sound is named a relevant sound), it triggers the recording of the next 6 seconds of audio data on the mobile device in the recording step. In other words, the detection of a relevant sound induces the recording of the 6 seconds audio signal located downstream of this relevant sound. In other words, to ensure the cough sound is fully covered, once exceeds the adaptive loudness threshold:

- 1. a cached buffer from previous frame is pre-appended;

- 2. an array of length = 6.5*16k (duration (seconds) times recording sample rate (kHz)) is then being filled up

- 3. if after the 6.5 seconds array is filled and immediately the next frame's max dB exceeds the theshold, the last frame of the written out array is pre-appended to current. Thus, they resulted in overlapping windows, with window size = 6.5 second and overlap size = 0.65 second

Subsequently, the processing step is executed in the smartphone. In the present invention, the audio signal that is recorded on the mobile device is filtered by the adaptative threshold.

In the present example, the processing step first comprises a preprocessing step that is constituted by:

- Applying a peak detection filter on the recorded segments to extract a determined number of peaks *per* recorded segment;

- Processing the extracted peaks of the patient's audio signal to provide the audio data;

In the present example, the peak detection filter selects up to 6 peaks per 6-second frame. Those peaks are selected in the following manner: From all peaks in the 6 seconds, that is all local maximum points, the peaks with the lowest amplitude will be discarded iteratively until the duration between any two of the remaining peaks is at a distance of at least 125ms. If the number of the remaining peaks is above 6, then the 6 peaks with the highest value will be selected, otherwise, if the number of the remaining peaks is 6 or lower, they will all be selected. The data used for further processing of these up to 6 peaks comprises a 650ms window centered around each peak (that is 325ms to the left/before and 325ms to the right/after).

Then the extracted peaks are processed as Mel spectrograms.

The Mel spectrograms are used as input for the symptom's detection steps based on trained inference models.

In the present example, the trained inference models that are used are described in the document in the document Barata et al, entitled "Automatic Recognition, Segmentation, and Sex Assignment of Nocturnal Asthmatic Coughs and Cough Epochs in Smartphone Audio Recordings: Observational Field Study", J.Med. Internet Res, vol.22, issue 7, 2020.

The trained inference models described in Barata et. Al, and used in the present example are 5 convolutional neural networks (CNN). These 5 CNNs have been trained on data collected during a longitudinal study. The collected data (sounds) were labeled either as a "cough" or as a "non-cough". Each of the model was trained on the same "cough" data, but on different "non-cough" data. The main features of these models are that they are lightweight and have been optimized for efficiency so that they could be run on processor-wise limited devices like smartphones. For example, these neural networks do not include fully connected layers, which, although common in most neural networks, are computationally expensive.

Overall, the detection steps based on the trained inference model comprises:

- i) Applying a first trained inference model on the Mel spectrograms to compute a probability as an output,

   said first trained inference model belonging to an ensemble of five trained inference models in the present example; and

   if the probability is inferior to a first reference value, here 90 % in the present example, the cough is rejected, whereas

   if the probability is superior to 90 %, step i) is repeated with the next trained inference model from said five trained inference model; and

- ii) the cough is validated if the probability is superior to 90 % (first reference value) for all the "five" trained models, and if there is a mean probability for all the "five" trained inference model of the ensemble that is above 98 % (as a second reference value) in the present example;

Then the detected cough is assigned to either male or female by using a computational tool. In this example, this is done using the 1) output of two Gaussian Mixture Models (GMM) that have been trained on the cough data from the study to identify male and female coughs and 2) using the information provided by the patient (their sex and whether or not they sleep alone) to determine the final output. That is, if the cough is assigned the same sex as the patient sex, then the cough is assigned to the patient. Otherwise, the cough assigned to a third person (partner for instance) and not counted.

Figure 2 is a scheme illustrating a second embodiment of the method for detecting patient's cough from audio data during an analyzing period of 7 hours during the night (patient asleep) and the day (patient awake) for instance in the present embodiment.

The monitoring is identical to the one of the first embodiment and will not be repeated herein.

In this second embodiment, the recording step comprises the recording of 6.5 second segment downstream the acoustic event whereas a 6 second segment was used in the first embodiment.

In this second embodiment, the recording step comprises the pre processing step is different from the one of the first embodiment. In this second embodiment, the peak filter comprises :

- Calculating the power (i.e. the sound level) of non overlapping time frames of 1500 samples *per* segment (6.5 seconds segment - 93.75 ms @ 16 kHz) as the root mean square of those samples;

- If a power of a frame is above a threshold of -50 dB), the power of said frame is compared to the power of the two preceding frames;

- If there is an increase in power of a threshold of-5 dB) (i.e. if the discrete curvature estimate is convex), the frame is considered as a frame of interest;

- the candidate peak amplitude needs to be above a multiple (currently 10, based on the M factor used for ALT) of the base noise level (mean of data).

- in these filtered frames of interest, the highest amplitudes are found and their indexes are the newfound peaks.

Then the extracted peaks are processed as Mel spectrograms.

The Mel spectrograms are used as input for the symptom's detection steps based on trained inference models.

In the second embodiment, the detection step is based on the 6 trained inference model:

- The five NN described in the first embodiment which are trained inference model for nighttime detection of acoustic event, here cough;

- 1 trained inference model trained for daytime cough detection.

The daytime trained inference model is applied to filter out the false positive acoustic event detected by the other 5 NN of the ensemble.

In this example, the symptom assignment is done using the 1) output of a model that has been trained on the cough data from the study to identify clear male, clear female, and gender unidentifiable coughs and 2) using customized rules derived from the evaluations and optionally the information provided by the patient (e.g., their age, diagnosis for rules, reference voice & sound samples, and their sex to assign) to determine the final output. That is, for example, for the latter, if the cough is detected as the opposite sex of the gender, this cough would be likely be assigned to a third person (partner for instance) and not counted.

In the second embodiment, the detection comprises:

- Increase symptom count by 1" after the Symptom detection validation;
- Then the Symptom assignment double-checks the counted cough, and if the counted cough does not have the same sex as the user (method describe here above), "Decrease symptom count by 1"

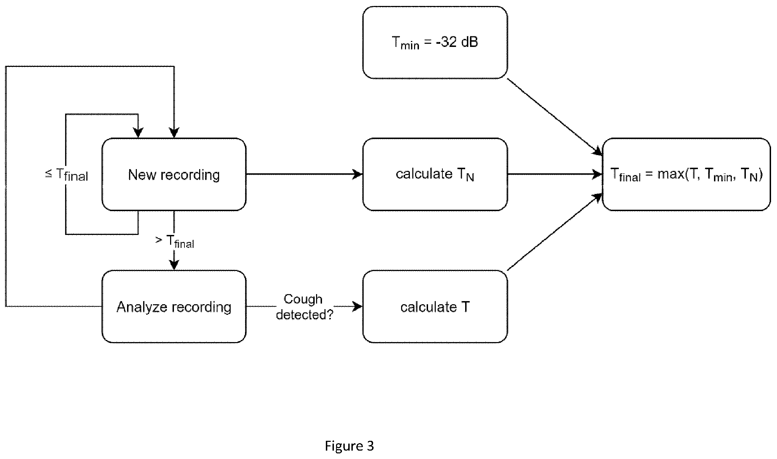

Figure 3 illustrates an example of the process to continuously adjust the adaptive loudness threshold according to the present invention.

In the present example, the adaptative loudness threshold is based on three components:

- a first parameter named T and based on the loudness of the detected acoustic events from the patient;

- a second parameter named T_N and based on a factor M times of the average loudness of the audio signal;

- a third parameter named T_min and based on a determined minimum threshold;

In the present example, the first parameter based on already detected coughs is determined as follows (the following being only an example of how this first parameter can be set, the invention is not limited to this example of first parameter T). The distribution of cough loudness is estimated as coughs are detected during the analyzing period and from that distribution the threshold T is set.

The estimated distribution comprises an initial distribution and an empirical distribution calculated from all coughs' loudness, each distribution being weighted and the weight varies during the analyzing period depends on the coughs detected. Starting with the initial distribution, the more coughs are detected, the greater the relative weight of the empirical distribution is.

The initial distribution itself comprises:

- a static initial distribution, for instance determined empirically from previous studies data, and that is preferably the same for all analyzing period and patients of one sex ; and

- a personal initial distribution, that is based on the estimated distributions of the preceding nights (analyzing period) for the patient

The initial distribution is a weighted average of the static distribution and the personal distribution.

The weight of the preceding nights' distribution can be 0 (zero) if not data from the preceding night (analyzing period) is available. Preferably, the weight will depend on the number of nights (analyzing period) the estimation has successfully taken place.

In the present example, the first parameter T is calculated by using the following formulas:

| | Symbol | Description | | Symbol | Description |
|---|---|---|---|---|---|
| | $T$ | Threshold based on cough loudness | | $\mu_0$ | Mean prior (from sample analysis) |
| | $\mu$ | Estimated mean | | $\sigma_0$ | Std prior (from sample analysis) |
| | $\sigma$ | Estimated std | | $\hat{\mu}$ | Empirical mean |
| | $L$ | Number of detected coughs | | $\hat{\sigma}$ | Empirical std |
| | $y^{(l)}$ | Amplitudes of detected coughs | | $\hat{V}$ | Empirical variance |

$$T = f(\mu, \sigma) = \mu - c_0 \cdot \sigma$$

where $c\_0 = 2.3$ is a tunable parameter The empirical values-are calculated during the analysis period as:

$$\hat{\mu} = \frac{1}{L}\sum_{l=1}^{L} y^{(l)} \qquad \text{and} \qquad \hat{V} = \frac{1}{L}\sum_{l=1}^{L}\left(y^{(l)}\right)^2 - \hat{\mu}^2$$

The estimated mean and variance will then be a weighted average of empirical values and prior:

$$\mu = a \cdot \hat{\mu} + b \cdot \mu_0 \qquad \text{and} \qquad \sigma = \sqrt{a \cdot \hat{V} + b \cdot \sigma_0^2} \qquad \text{with} \qquad a + b = 1$$

The estimated values tend away from the priors to the empirical ones as the number of samples increases in the following manner:

$$a = 1 - e^{-rL} \qquad \text{and} \qquad b = e^{-rL}$$

where $r = 0.1$ is a tunable parameter

In the present example, the second parameter T_N is based on the noise level said noise level being the average loudness in at a time t of an analysis period. T_N is a factor M times said noise level.

In the present example, T_N = -26 dB; M=10. The formula for converting an amplitude A (like 0.005) to dB is 20*log10(A). This is a common definition. A mathematical property of the log function in there is that log10(10*A) equals log10(A) + 1. That is why a 10 times higher amplitude, is 20dB higher.

For instance, if the average loudness (noise) is Amplitude = 0.005 (=corresponding to -46dB) then T_N = 10*0.005 = 0.05 (corresponding to = -26dB).

In the present example, the third parameter T_min is a static parameter corresponding to the minimum loudness threshold, here T_min = -32 dB.

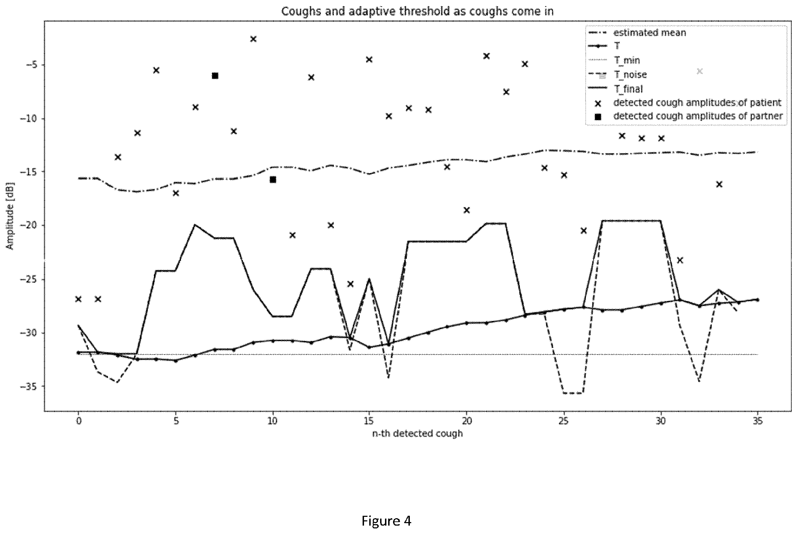

The present invention is further illustrated by three examples supported respectively by figure 4, figure 5 and figure 6.

The three examples, the point is to compare the detected cough by using either the method according to the present invention represented and explained on figure 1 and corresponding detail here above, or the method described in Barata et al. ("Automatic Recognition, Segmentation, and Sex Assignment of Nocturnal Asthmatic Coughs and Cough Epochs in Smartphone Audio Recordings: Observational Field Study", J.Med. Internet Res, vol.22, issue 7, 2020).

Comparison example 1:

In example 1, the experience is based on the following parameters:

- analyzing period = 4 hours during the night

- patient: one patient and one partner (two distinct persons)

- environment: one patient and one partner in a shared bedroom (note: Partner coughs do not influence the adaptative loudness threshold).

The results of the comparison example 1 is illustrated on table 1 below.

TABLE 1:

|  | Inferences run | Coughs detected |
| --- | --- | --- |
| *Adaptive threshold* | 355 | 35 / 36 |
| *Static threshold -26dB* | 371 | 33/36 |
| *Barata* | 1040 | 13/36 |

An inference is an application of any of the 5 trained NN models. To process one frame fully (to the point of cough detection) would require 5 inferences. However, if the application of the first model would already give a probability below the first reference value, this frame is discarded and thus would only have run one inference.

Adapting loudness threshold (table 1, figure 4):

In this first example, the adaptive threshold starts at around -32dB and tends to around -24.5dB. The total number of frames processed is only slightly higher than with Barata but almost all coughs are detected instead of around a third with Barata's. Also, the adaptive threshold processes slightly fewer peaks and find slightly more coughs than with a static threshold. Since Barata also always uses all five models, the number of inferences he runs is way higher. Overall, the adaptive threshold is mostly governed by the noise threshold $T\_N$ (i.e., second parameter) and the cough amplitude-based threshold (i.e., first parameter).

Cough detection (table 1, figure 4):

The true number of coughs in this first example is 36, of which the adaptive thresholding detects 35 using 215 processing steps (see figure 1). The number of coughs detected (=35) is significantly higher compared to the number of coughs detected by Barata's (13).

Comparison of example 2:

Example 2:

In example 2, the experience is based on the following parameters:

- analyzing period = 4 hours during the night

- patient: one patient and one partner (two distinct persons)

- environment: one patient and one partner in a shared bedroom (note: Partner coughs do not influence the adaptative loudness threshold).

The results of the comparison example 2 is illustrated on table 2 below.

TABLE 2:

|  | Inferences run | Coughs detected |
| --- | --- | --- |
| *Adaptive threshold* | 1583 | 296 / 444 |
| *Static threshold -26dB* | 1174 | 204 / 444 |
| *Barata* | 1620 | 107 / 444 |

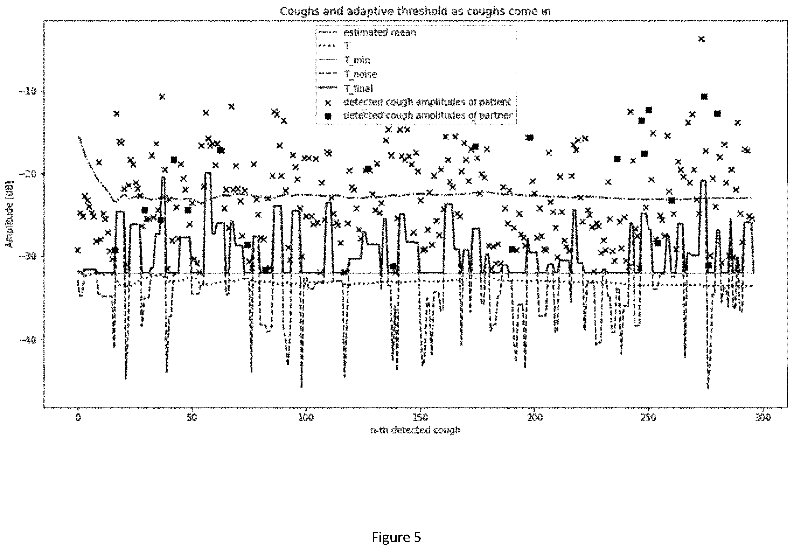

Adapting loudness threshold (table 2, figure 5)

In this example the adaptive threshold starts at around -32dB and pretty much stays there (except for noisy periods). The total number of frames processed is still higher than with Barata,

Cough detection (table 2, figure 5):

However, the adaptive threshold is able to detect significantly many more coughs, that would have gone unnoticed with Barata. The static threshold is also much better than Barata, however still far below the adaptive threshold in terms of coughs detected.

Comparison example 3:

In example 3, the experience is based on the following parameters:

- analyzing period = 1 hour during the day

- patient: One patient

- environment:. One patient monitoring during the day, with a very high level of background noise and other loud sounds (possibly multiple distinct persons around).

The results of the comparison example 3 is illustrated on table 3 below.

TABLE 3:

|  | Inferences run | Coughs detected |
|---|---|---|
| *Adaptive threshold* | 102 | 0 / 0 |
| *Static threshold -26dB* | 3191 | 0 / 0 |
| *Barata* | 29570 | 0 / 0 |

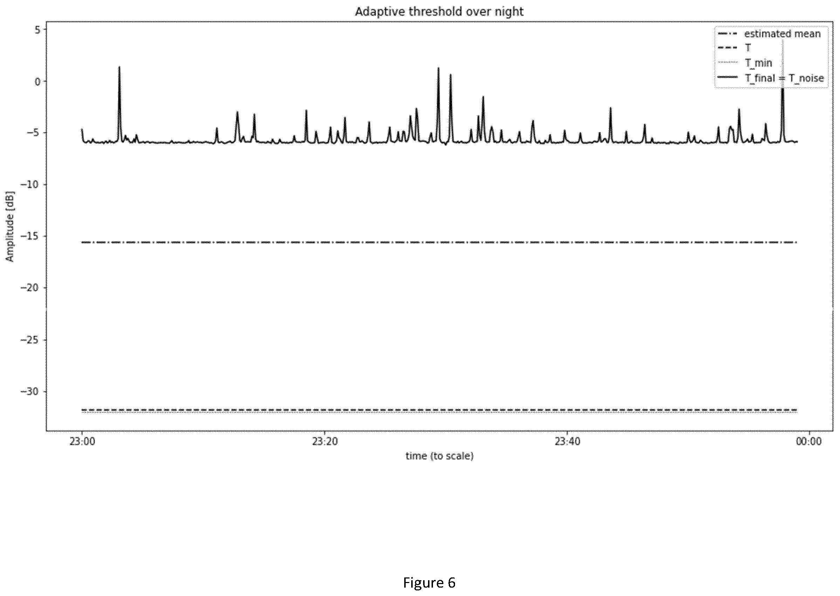

Adapting loudness threshold (table 3, figure 6)

The adaptive threshold is mostly governed by the noise threshold and the minimum threshold. The coughs are rather silent and hard to detect. In this noisy night (for example it could be that the patient did not go to sleep, but had a night out) were no coughs occurred the adaptive threshold shows its strength mainly due to noise exclusion. Both with Barata and with the static threshold thousands of frames would be processed for nothing. The adaptive threshold would process only 102.

Cough detection (table 2, figure 5):

The true number of coughs in this first example is 444, of which the adaptive thresholding detects 296 using 399 processing steps (preprocessing and neural network). The adaptive threshold is mostly governed by the noise threshold and the minimum threshold. The coughs are rather silent and hard to detect.
While the embodiments have been described in conjunction with a number of embodiments, it is evident that many alternatives, modifications and variations would be or are apparent to those of ordinary skill in the applicable arts. Accordingly, this disclosure is intended to embrace all such alternatives, modifications, equivalents and variations that are within the scope of this disclosure. This for example particularly the case regarding the different apparatuses which can be used. The invention is defined in the appended claims 1- 20.

**Claims**

1. Computer-implemented method for detecting a patient's acoustic events, for instance coughs, from audio data of the patient during an analyzing period, the method comprising:

    - a recording step to record audio signals from the patient by using a mobile device comprising recording means, and
    - a processing step to process the recorded audio signals into audio data by using a processing module embedded in the device,
    - a detection step to detect said acoustic events from the audio data,
    wherein the method further comprises a monitoring step, before the recording step, to select segments of a determined duration of the patient's audio signal to be recorded on the device, said monitoring step comprising:

- applying a threshold on the patient's audio signal to identify sounds that exceed said threshold named relevant sounds, and
- recording on the device the segments of the patient's audio signal located downstream said identified relevant sounds,

**characterized in that** the threshold is an adaptive loudness threshold named ALT, the adaptative loudness threshold being based on an initial loudness threshold that is configured to be adjusted continuously during said analyzing period and is adjusted based on at least three parameters defined as:

- a first parameter named T and based on the loudness of the detected acoustic events from the patient;
- a second parameter named T_N and based on a factor M times of the average loudness of the audio signal;
- a third parameter named T_min and based on a determined minimum threshold;

wherein said adaptative loudness threshold ALT corresponds to the maximum value chosen among the first parameter, the second parameter and the third parameter and is defined as

$$ALT = \max(T, T\_min, T\_N).$$

2. Method according to claim 1, wherein the first parameter T is based on the distribution of the acoustic event detected in the patient during the analyzing period, said distribution being adjusted continuously as the acoustic events are detected.

3. Method according to claims 1 or 2, wherein the factor M time of the second parameter T_N is chosen between 0 and about 100, preferably about 2 and about 50, more preferably between about 4 and about 25, in particular between about 8 and about 12.5, for instance between about 9 and about 11, for instance 10.

4. Method according to any one of claims 1 to 3, wherein the minimum threshold of the third parameter is comprised between about -18dB and about -45dB, preferably between about -22dB and about -42dB, more preferably between about -27dB and about -37dB, in particular between about -30dB and about -34dB, for instance between about -31dB and about -33dB, for instance about -32dB.

5. Method according to any one of claims 1 to 4, wherein the detection step comprises applying an ensemble of at least one trained inference model on said recorded segment of the audio data of the patient as an input to compute a probability as an output to detect an acoustic event.

6. Method according to claim 5, wherein said ensemble comprising one trained inference model, the detection step comprises;

- i) Applying said inference model on said recorded segment of audio data of the patient as an input, to compute a probability as an output, said audio data contains at least one acoustic event,
- ii) if the probability is inferior to a reference value the acoustic event is rejected, whereas if the probability is superior to the reference value the acoustic event is validated;

7. Method according to any one of claims 1 to 6, wherein the detection step comprises;

- i) Applying a first trained inference model on said recorded segment of audio data of the patient as an input, to compute a probability as an output,

said audio data contains at least one acoustic event,
said first trained inference model belonging to an ensemble of trained inference models comprising "n" trained inference models, "n" superior or equal to two, to detect said acoustic events; and
if the probability is inferior to a first reference value, the acoustic event is rejected, whereas
if the probability is superior to the first reference value, step i) is repeated with the next trained inference model from said ensemble comprising "n" trained inference model; and

- ii) the acoustic event is validated if the probability is superior to the first reference value for all the "n" trained models, and if there is a mean probability for all the "n" trained inference model of the ensemble that is above a second reference value;

8. Method according to claim 7, wherein the first reference value is correlated with the mean probability for all the inference model of the ensemble and the number "n" of trained inference models by using the following formula:

$$\text{First reference value} = 1 - ((1 - \text{second reference value}) \times \text{number "n"})$$

9. Method according to any one of claims 6 to 8, wherein at least one of the "n" trained inference models of the ensemble is dedicated to the detection of a type of patient's acoustic events, for instance daytime acoustic event, dry or wet cough, wheeze cough, snoring, specific breathing patterns.

10. Method according to any one of claims 6 to 9, wherein at least one of "n" trained inference models of the ensemble is dedicated to the detection of daytime acoustic event.

11. Method according to any one of claims 6 to 10, wherein at least one of "n" trained inference models of the ensemble is dedicated to the detection of nighttime acoustic event.

12. Method according to any one of claims 6 to 11, wherein the "n" ensemble of trained inference model comprises one trained inference model, preferably said trained inference model being trained for detecting acoustic event in day time and in night time.

13. Method according to any one of claims 1 to 12, wherein the processing step comprises a pre-processing step that comprises:

    - Applying a peak detection filter on said recorded segments to extract a determined number of peaks per recorded segment;
    - Processing the extracted peaks of the patient's audio signal to provide the audio data;

14. Method according to claim 13, wherein the number of peak detectable per recorded segment is adaptive and is determined depending on the processing module specifications, for instance the processing speed or the calculation power of said processing module or acoustic environment.

15. Method according to any one of claims 1 to 14, wherein the method further comprises, before the monitoring step, a status check step comprising:

    - determining if the device is in use or not in use based on at least one sensor of the device, and starting the monitoring step if the device is not in use; and/or
    - determining if the device has an energy level above a predetermined level, and starting the monitoring step if the energy level is above the predetermined level;

16. Method according to the preceding claim, wherein the status check is based on sensors installed in the device capable of giving information on the position of the device, for instance an accelerometer, and/or on the status of the device, for instance, screen brightness, battery status.

17. Method according to any one of claims 1 to 16, wherein the patient is comprised in a group of "p" patients comprising at least two patients, the method further comprising an assigning step for assigning a detected acoustic event to a patient from the said group, the assigning step comprising:

    - Applying a computational tool, for instance a statistical model on the detected acoustic event, said computational tool being configured to assign a detected acoustic event to a predetermined patient;
    - Attributing the detected acoustic event to one patient from the group if there is a correlation between said computation tool and the detected acoustic event;

18. Method according to claim 17, wherein

either the computation tool has been trained to assign a detected acoustic event to a type of patient is based on gender, for instance male or female, age for instance child or adult;

or the computation tool has been trained to assign a detected acoustic event to a designated patient based on previously detected acoustic event from said designated patient.

19. Device comprising means for carrying out the method of any one of claims 1 to 18 preferably the device is chosen among a mobile device, for instance a smartphone.

20. Computer program comprising instructions which, when the program is executed by a computer, cause the computer to carry out the method of any one of claims 1 to 18.

**Patentansprüche**

1. Computerimplementiertes Verfahren zur Erkennung von akustischen Ereignissen eines Patienten, beispielsweise Husten, anhand von Audiodaten des Patienten während eines Analysezeitraums, wobei das Verfahren umfasst:

   - einen Aufzeichnungsschritt zum Aufzeichnen von Audiosignalen des Patienten unter Verwendung einer mobilen Vorrichtung, das Aufzeichnungsmittel umfasst, und
   - einen Verarbeitungsschritt zum Verarbeiten der aufgezeichneten Audiosignale zu Audiodaten unter Verwendung eines in die Vorrichtung eingebetteten Verarbeitungsmoduls,
   - einen Erkennungsschritt zum Erkennen der akustischen Ereignisse aus den Audiodaten,
   wobei das Verfahren ferner einen Überwachungsschritt vor dem Aufzeichnungsschritt umfasst, um Segmente einer bestimmten Dauer des Audiosignals des Patienten auszuwählen, die auf der Vorrichtung aufgezeichnet werden sollen, wobei der Überwachungsschritt umfasst:

   - Anwenden eines Schwellenwerts auf das Audiosignal des Patienten, um Geräusche zu identifizieren, die diesen Schwellenwert überschreiten, die als relevante Geräusche bezeichnet werden, und
   - Aufzeichnen der Segmente des Audiosignals des Patienten, die sich den identifizierten relevanten Geräuschen nachgelagert befinden, auf der Vorrichtung,

   **dadurch gekennzeichnet, dass** der Schwellenwert ein adaptiver Lautstärkeschwellenwert ist, der als ALT bezeichnet wird, wobei der adaptive Lautstärkeschwellenwert auf einem anfänglichen Lautstärkeschwellenwert basiert, der so ausgestaltet ist, dass er während des Analysezeitraums kontinuierlich angepasst wird, und auf mindestens drei Parametern beruht, die wie folgt definiert sind:

   - einen ersten Parameter, der mit T bezeichnet wird, der auf der Lautstärke der von dem Patienten erfassten akustischen Ereignisse basiert;
   - einen zweiten Parameter, der mit T_N bezeichnet wird, der auf dem Faktor M mal die durchschnittliche Lautstärke des Audiosignals basiert;
   - einen dritten Parameter, der als T_min bezeichnet wird, der auf einem festgelegten Mindestschwellenwert basiert;

   wobei der adaptive Lautstärkeschwellenwert ALT dem Maximalwert entspricht, der aus dem ersten Parameter, dem zweiten Parameter und dem dritten Parameter ausgewählt wird, und definiert ist als

$$ALT = \max(T, T\_min, T\_N).$$

2. Verfahren nach Anspruch 1, wobei der erste Parameter T auf der Verteilung der während des Analysezeitraums bei dem Patienten erfassten akustischen Ereignisse basiert, wobei diese Verteilung kontinuierlich angepasst wird, während die akustischen Ereignisse erfasst werden.

3. Verfahren nach Anspruch 1 oder 2, wobei der Faktor M des zweiten Parameters T_N zwischen 0 und etwa 100, vorzugsweise zwischen etwa 2 und etwa 50, noch bevorzugter zwischen etwa 4 und etwa 25, insbesondere zwischen etwa 8 und etwa 12,5, beispielsweise zwischen etwa 9 und etwa 11, beispielsweise 10, ausgewählt wird.

4. Verfahren nach einem der Ansprüche 1 bis 3, wobei der Mindestschwellenwert des dritten Parameters zwischen etwa -18 dB und etwa -45 dB, vorzugsweise zwischen etwa -22 dB und etwa -42 dB, noch bevorzugter zwischen etwa -27 dB und etwa -37 dB, insbesondere zwischen etwa -30 dB und etwa -34 dB, beispielsweise zwischen etwa -31 dB und etwa -33 dB, beispielsweise etwa -32 dB liegt.

5. Verfahren nach einem der Ansprüche 1 bis 4, wobei der Erkennungsschritt das Anwenden einer Anordnung aus mindestens einem trainierten Inferenzmodell auf das aufgezeichnete Segment der Audiodaten des Patienten als Eingabe umfasst, um eine Wahrscheinlichkeit als Ausgabe zur Erkennung eines akustischen Ereignisses zu berechnen.

6. Verfahren nach Anspruch 5, wobei die Anordnung ein trainiertes Inferenzmodell umfasst und der Erkennungsschritt umfasst:

   - i) Anwenden des Inferenzmodells auf das aufgezeichnete Segment der Audiodaten des Patienten als Eingabe, um eine Wahrscheinlichkeit als Ausgabe zu berechnen, wobei die Audiodaten mindestens ein akustisches Ereignis enthalten,
   - ii) wobei, wenn die Wahrscheinlichkeit unter einem Referenzwert liegt, das akustische Ereignis verworfen wird, während hingegen das akustische Ereignis als valide eingestuft wird, wenn die Wahrscheinlichkeit über dem Referenzwert liegt;

7. Verfahren nach einem der Ansprüche 1 bis 6, wobei der Erkennungsschritt umfasst:

   - i) Anwenden eines ersten trainierten Inferenzmodells auf das aufgezeichnete Segment von Audiodaten des Patienten als Eingabe, um eine Wahrscheinlichkeit als Ausgabe zu berechnen,

     wobei die Audiodaten mindestens ein akustisches Ereignis enthalten,
     wobei das erste trainierte Inferenzmodell zu einer Anordnung aus trainierten Inferenzmodellen gehört, die "n" trainierte Inferenzmodelle umfasst, wobei "n" größer oder gleich zwei ist, um die akustischen Ereignisse zu erkennen; und
     wenn die Wahrscheinlichkeit kleiner als ein erster Referenzwert ist, das akustische Ereignis verworfen wird, während hingegen
     wenn die Wahrscheinlichkeit größer als der erste Referenzwert ist, Schritt i) mit dem nächsten trainierten Inferenzmodell aus der Anordnung wiederholt wird, die "n" trainierte Inferenzmodelle umfasst; und

   - ii) das akustische Ereignis als valide eingestuft wird, wenn die Wahrscheinlichkeit bei allen "n" trainierten Modellen über dem ersten Referenzwert liegt und wenn die mittlere Wahrscheinlichkeit aller "n" trainierten Inferenzmodelle der Anordnung über einem zweiten Referenzwert liegt;

8. Verfahren nach Anspruch 7, wobei der erste Referenzwert mit der mittleren Wahrscheinlichkeit für alle Inferenzmodelle der Anordnung und der Anzahl "n" der trainierten Inferenzmodelle unter Verwendung der folgenden Formel korreliert wird:

$$\text{Erster Referenzwert} = 1 - ((1 - \text{zweiter Referenzwert}) \times \text{Anzahl „n"})$$

9. Verfahren nach einem der Ansprüche 6 bis 8, wobei mindestens eines der "n" trainierten Inferenzmodelle der Anordnung für die Erkennung einer Art von akustischen Ereignissen des Patienten dediziert ist, beispielsweise eines akustischen Ereignisses am Tag, eines trockenen oder feuchten Hustens, eines pfeifenden Hustens, des Schnarchens oder spezifischer Atemmuster.

10. Verfahren nach einem der Ansprüche 6 bis 9, wobei mindestens eines der "n" trainierten Inferenzmodelle der Anordnung für die Erkennung von akustischen Ereignissen am Tag dediziert ist.

11. Verfahren nach einem der Ansprüche 6 bis 10, wobei mindestens eines der "n" trainierten Inferenzmodelle des Ensembles für die Erkennung von nächtlichen akustischen Ereignissen dediziert ist.

12. Verfahren nach einem der Ansprüche 6 bis 11, wobei das "n"-Ensemble aus trainierten Inferenzmodellen ein trainiertes Inferenzmodell umfasst, wobei das trainierte Inferenzmodell vorzugsweise für die Erkennung akustischer Ereignisse tagsüber und nachts trainiert ist.

**13.** Verfahren nach einem der Ansprüche 1 bis 12, wobei der Verarbeitungsschritt einen Vorverarbeitungsschritt umfasst, der Folgendes umfasst:

- Anwenden eines Spitzenerkennungsfilters auf die aufgezeichneten Segmente, um eine bestimmte Anzahl von Spitzen *pro* aufgezeichnetem Segment zu extrahieren;
- Verarbeiten der extrahierten Spitzen des Audiosignals des Patienten, um die Audiodaten bereitzustellen;

**14.** Verfahren nach Anspruch 13, wobei die Anzahl der *pro* aufgezeichnetem Segment erfassbaren Spitzen adaptiv ist und in Abhängigkeit von den Spezifikationen des Verarbeitungsmoduls, beispielsweise der Verarbeitungsgeschwindigkeit oder der Rechenleistung des Verarbeitungsmoduls, oder der akustischen Umgebung bestimmt wird.

**15.** Verfahren nach einem der Ansprüche 1 bis 14, wobei das Verfahren vor dem Überwachungsschritt ferner einen Statusprüfungsschritt umfasst, der Folgendes umfasst:

- Feststellen, ob die Vorrichtung in Verwendung ist oder nicht, basierend auf mindestens einem Sensor der Vorrichtung, und Starten des Überwachungsschritts, wenn die Vorrichtung nicht in Verwendung ist; und/oder
- Feststellen, ob die Vorrichtung einen Energiepegel aufweist, der über einem vorgegebenen Pegel liegt, und Starten des Überwachungsschritts, wenn der Energiepegel über dem vorgegebenen Pegel liegt;

**16.** Verfahren nach dem vorhergehenden Anspruch, wobei die Statusprüfung auf in der Vorrichtung installierten Sensoren basiert, die Informationen über die Position der Vorrichtung liefern können, beispielsweise einem Beschleunigungsmesser, und/oder über den Status der Vorrichtung, zum Beispiel die Bildschirmhelligkeit oder den Batteriestatus.

**17.** Verfahren nach einem der Ansprüche 1 bis 16, wobei der Patient zu einer Gruppe von "p" Patienten gehört, die mindestens zwei Patienten umfasst, wobei das Verfahren ferner einen Zuordnungsschritt zum Zuordnen eines erfassten akustischen Ereignisses zu einem Patienten aus der genannten Gruppe umfasst, wobei der Zuordnungsschritt umfasst:

- Anwenden eines Berechnungswerkzeugs, beispielsweise eines statistischen Modells, auf das erfasste akustische Ereignis, wobei das Berechnungswerkzeug so ausgestaltet ist, dass es ein erfasstes akustisches Ereignis einem vorbestimmten Patienten zuordnet;
- Zuweisen des erfassten akustischen Ereignisses zu einem Patienten aus der Gruppe, wenn eine Korrelation zwischen dem Berechnungswerkzeug und dem erfassten akustischen Ereignis besteht;

**18.** Verfahren nach Anspruch 17, wobei

entweder das Berechnungswerkzeug darauf trainiert wurde, ein erfasstes akustisches Ereignis einem Patiententyp zuzuordnen, der auf dem Geschlecht, beispielsweise männlich oder weiblich, oder dem Alter, beispielsweise Kind oder Erwachsener, basiert;
oder das Berechnungswerkzeug darauf trainiert wurde, ein erfasstes akustisches Ereignis einem bestimmten Patienten auf der Grundlage eines zuvor erfassten akustischen Ereignisses von diesem bestimmten Patienten zuzuordnen.

**19.** Vorrichtung, die Mittel zur Durchführung des Verfahrens nach einem der Ansprüche 1 bis 18 umfasst, wobei die Vorrichtung vorzugsweise aus dem Kreis der mobilen Vorrichtungen, beispielsweise einem Smartphone, ausgewählt ist.

**20.** Computerprogramm, das Anweisungen umfasst, die, wenn das Programm von einem Computer ausgeführt wird, den Computer veranlassen, das Verfahren nach einem der Ansprüche 1 bis 18 durchzuführen.

**Revendications**

**1.** Procédé de mise en œuvre par ordinateur, destiné à détecter des événements acoustiques d'un patient, par exemple des toux, à partir de données audios du patient pendant une période d'analyse, le procédé comprenant :

- une étape d'enregistrement consistant à enregistrer des signaux audios provenant du patient à l'aide d'un dispositif mobile comprenant des moyens d'enregistrement, et
- une étape de traitement pour traiter les signaux audios enregistrés en données audio à l'aide d'un module de traitement intégré dans le dispositif,
- une étape de détection pour détecter lesdits événements acoustiques à partir des données audios,
dans laquelle le procédé comprend en outre une étape de surveillance, avant l'étape d'enregistrement, pour sélectionner des segments d'une durée déterminée du signal audio du patient à enregistrer sur le dispositif, ladite étape de surveillance comprenant :

- l'application d'un seuil au signal audio du patient pour identifier les sons qui dépassent ledit seuil, appelés sons pertinents, et
- l'enregistrement sur le dispositif des segments du signal audio du patient situés en aval desdits sons pertinents identifiés,

**caractérisé en ce que** le seuil est un seuil de volume adaptatif dénommé ALT, ce seuil de volume adaptatif étant basé sur un seuil de volume initial configuré pour être ajusté en continu pendant ladite période d'analyse et reposant sur au moins trois paramètres définis comme suit :

- un premier paramètre appelé T, basé sur l'intensité sonore des événements acoustiques détectés chez le patient ;
- un deuxième paramètre nommé T_N et basé sur un facteur M fois l'intensité sonore moyenne du signal audio ;
- un troisième paramètre nommé T_min et basé sur un seuil minimal déterminé ;

dans lequel ledit seuil d'intensité sonore adaptatif ALT correspond à la valeur maximale choisie parmi le premier paramètre, le deuxième paramètre et le troisième paramètre et est défini comme suit

$$ALT = max\ (T,\ T\_min,\ T\_N).$$

2. Procédé selon la revendication 1, dans lequel le premier paramètre T est basé sur la distribution des événements acoustiques détectés chez le patient pendant la période d'analyse, ladite distribution étant ajustée en continu au fur et à mesure que les événements acoustiques sont détectés.

3. Procédé selon les revendications 1 ou 2, dans lequel le facteur M du deuxième paramètre T_N est choisi entre 0 et environ 100, de préférence entre environ 2 et environ 50, plus préférablement entre environ 4 et environ 25, en particulier entre environ 8 et environ 12,5, par exemple entre environ 9 et environ 11, par exemple 10.

4. Procédé selon l'une quelconque des revendications 1 à 3, dans lequel le seuil minimal du troisième paramètre est compris entre environ -18 dB et environ -45 dB, de préférence entre environ -22 dB et environ -42 dB, plus préférablement entre environ -27 dB et environ -37 dB, en particulier entre environ -30 dB et environ -34 dB, par exemple entre environ -31 dB et environ -33 dB, par exemple environ -32 dB.

5. Procédé selon l'une quelconque des revendications 1 à 4, dans lequel l'étape de détection comprend l'application d'un ensemble d'au moins un modèle d'inférence entraîné sur ledit segment enregistré des données audio du patient en tant qu'entrée pour calculer une probabilité en tant que sortie afin de détecter un événement acoustique.

6. Procédé selon la revendication 5, dans lequel ledit ensemble comprend un modèle d'inférence entraîné, l'étape de détection comprenant :

- i) l'application dudit modèle d'inférence audit segment enregistré de données audio du patient en tant qu'entrée, pour calculer une probabilité en tant que sortie, lesdites données audio contenant au moins un événement acoustique,
- ii) si la probabilité est inférieure à une valeur de référence, l'événement acoustique est rejeté, tandis que si la probabilité est supérieure à la valeur de référence, l'événement acoustique est validé ;

7. Procédé selon l'une quelconque des revendications 1 à 6, dans lequel l'étape de détection comprend :

- i) l'application d'un premier modèle d'inférence entraîné sur ledit segment enregistré de données audio du patient en tant qu'entrée, afin de calculer une probabilité en tant que sortie,

> lesdites données audios contiennent au moins un événement acoustique,
> ledit premier modèle d'inférence entraîné appartenant à un ensemble de modèles d'inférence entraînés comprenant « n » modèles d'inférence entraînés, « n » étant supérieur ou égal à deux, pour détecter lesdits événements acoustiques ; et
> si la probabilité est inférieure à une première valeur de référence, l'événement acoustique est rejeté, tandis que
> si la probabilité est supérieure à la première valeur de référence, l'étape i) est répétée avec le modèle d'inférence entraîné suivant dudit ensemble comprenant « n » modèles d'inférence entraînés ; et

> - ii) l'événement acoustique est validé si la probabilité est supérieure à la première valeur de référence pour l'ensemble des « n » modèles entraînés, et si la probabilité moyenne pour l'ensemble des « n » modèles d'inférence entraînés de l'ensemble est supérieure à une deuxième valeur de référence ;

8. Procédé selon la revendication 7, dans lequel la première valeur de référence est corrélée à la probabilité moyenne pour tous les modèles d'inférence de l'ensemble et au nombre « n » de modèles d'inférence entraînés à l'aide de la formule suivante :

Première valeur de référence = 1 - ((1 - deuxième valeur de référence) $\times$ nombre « n »)

9. Procédé selon l'une quelconque des revendications 6 à 8, dans lequel au moins l'un des « n » modèles d'inférence entraînés de l'ensemble est dédié à la détection d'un type d'événements acoustiques du patient, par exemple un événement acoustique diurne, une toux sèche ou grasse, une toux sifflante, des ronflements, des schémas respiratoires spécifiques.

10. Procédé selon l'une quelconque des revendications 6 à 9, dans lequel au moins l'un des « n » modèles d'inférence entraînés de l'ensemble est dédié à la détection d'événements acoustiques diurnes.

11. Procédé selon l'une quelconque des revendications 6 à 10, dans lequel au moins l'un des « n » modèles d'inférence entraînés de l'ensemble est dédié à la détection d'événements acoustiques nocturnes.

12. Procédé selon l'une quelconque des revendications 6 à 11, dans lequel l'ensemble « n » de modèles d'inférence entraînés comprend un modèle d'inférence entraîné, ledit modèle d'inférence entraîné étant de préférence entraîné pour détecter des événements acoustiques pendant la journée et pendant la nuit.

13. Procédé selon l'une quelconque des revendications 1 à 12, dans lequel l'étape de traitement comprend une étape de prétraitement qui comprend :

> - l'application d'un filtre de détection de pics sur lesdits segments enregistrés afin d'extraire un nombre déterminé de pics *par* segment enregistré ;
> - le traitement des pics extraits du signal audio du patient pour fournir les données audios ;

14. Procédé selon la revendication 13, dans lequel le nombre de pics détectables *par* segment enregistré est adaptatif et est déterminé en fonction des spécifications du module de traitement, par exemple la vitesse de traitement ou la puissance de calcul dudit module de traitement, ou de l'environnement acoustique.

15. Procédé selon l'une quelconque des revendications 1 à 14, dans lequel le procédé comprend en outre, avant l'étape de surveillance, une étape de vérification de l'état comprenant :

> - déterminer si le dispositif est en cours d'utilisation ou non sur la base d'au moins un capteur du dispositif, et lancer l'étape de surveillance si le dispositif n'est pas en cours d'utilisation ; et/ou
> - déterminer si le dispositif présente un niveau d'énergie supérieur à un niveau prédéterminé, et lancer l'étape de surveillance si le niveau d'énergie est supérieur au niveau prédéterminé ;

16. Procédé selon la revendication précédente, dans lequel la vérification d'état est basée sur des capteurs installés dans le dispositif, capables de fournir des informations sur la position du dispositif, par exemple un accéléromètre, et/ou sur l'état du dispositif, par exemple la luminosité de l'écran, l'état de la batterie.

17. Procédé selon l'une quelconque des revendications 1 à 16, dans lequel le patient fait partie d'un groupe de « p » patients comprenant au moins deux patients, le procédé comprenant en outre une étape d'attribution pour attribuer un événement acoustique détecté à un patient dudit groupe, l'étape d'attribution comprenant :

- l'application d'un outil de calcul, par exemple un modèle statistique, à l'événement acoustique détecté, ledit outil de calcul étant configuré pour attribuer un événement acoustique détecté à un patient prédéterminé ;
- l'attribution de l'événement acoustique détecté à un patient du groupe s'il existe une corrélation entre ledit outil de calcul et l'événement acoustique détecté ;

18. Procédé selon la revendication 17, dans lequel

soit l'outil de calcul a été entraîné pour attribuer un événement acoustique détecté à un type de patient en fonction du sexe, par exemple un homme ou une femme, ou de l'âge, par exemple un enfant ou un adulte ;
soit l'outil de calcul a été entraîné pour attribuer un événement acoustique détecté à un patient désigné sur la base d'un événement acoustique précédemment détecté chez ledit patient désigné.

19. Dispositif comprenant des moyens pour mettre en œuvre le procédé selon l'une quelconque des revendications 1 à 18, de préférence le dispositif est choisi parmi un dispositif mobile, par exemple un smartphone.

20. Programme informatique comprenant des instructions qui, lorsque le programme est exécuté par un ordinateur, amènent l'ordinateur à mettre en œuvre le procédé selon l'une quelconque des revendications 1 à 18.

Figure 1

Figure 2

EP 4 362 805 B1

Figure 3

Figure 4

Figure 5

EP 4 362 805 B1

Figure 6

Sound

Cough model 1

Cough model 2

Cough model 3

Cough model 4

Cough model 5

Average probability

Decision cough: yes/no

Figure 7

Sound

Cough model

Snoring model

Decision cough: yes/no

Decision snoring: yes/no

Wet cough model

Decision wet cough: yes/no

Figure 8

## REFERENCES CITED IN THE DESCRIPTION

**Patent documents cited in the description**

- US 2015245788 A

**Non-patent literature cited in the description**

- **BARATA et al.** Automatic Recognition, Segmentation, and Sex Assignment of Nocturnal Asthmatic Coughs and Cough Epochs in Smartphone Audio Recordings: Observational Field Study. *J.Med. Internet Res*, 2020, vol. 22 (7)